# EUROPEAN PATENT APPLICATION

(11) **EP 4 451 233 A1**
(43) Date of publication of application: **23.10.2024**
(21) Application number: 24168852.2
(22) Date of filing: 05.04.2024
(51) Int. Cl.: G06V 20/40, H04N 25/47

(54) **NEUROMORPHIC SENSORS FOR LOW POWER WEARABLES**

(30) Priority: 19.04.2023 US 202318136583
(71) Applicant: Rockwell Collins, Inc., Cedar Rapids, IA 52498 (US)
(72) Inventor: LORE, Kin Gwn, East Hartford, CT (US); SUNDARAMOORTHI, Ganesh, East Hartford, CT (US); REDDY, Kishore K., East Hartford, CT (US)
(74) Representative: Dehns

(57) **Abstract**

A wearable device includes neuromorphic event cameras (104). A processor (100) receives data streams from the event cameras (104) and makes application specific predictions / determinations. The event cameras (104) may be outward facing to make determinations about the environment or a specific task, inward facing to monitor the state of the user, or both. The processor (100) may be configured as a trained neural network to receive the data streams and produce output based on predefined sets of training data. Sensors other than event cameras (104) may supply data to the processor (100) and neural network, including other cameras (104) via a feature recognition process.

## Description

### BACKGROUND

Egocentric cameras are used in wearables to monitor the behavior of users (e.g., technicians, pilots, warfighters, etc.) for efficiency, lifestyle, and health monitoring purposes. The cameras have very low frame rate and are battery intensive. Low frame rate causes adjacent images to have significant appearance changes, so motion cannot be reliably estimated. When embodied in wearables, the motion of the wearer's head combined with the low frame rate results in significant motion blur.

### SUMMARY

In one aspect, embodiments of the inventive concepts disclosed herein are directed to a wearable device with neuromorphic event cameras. A processor receives data streams from the event cameras and makes application specific predictions / determinations. The event cameras may be outward facing to make determinations about the environment or a specific task, inward facing to monitor the state of the user, or both.

In a further aspect, the processor may be configured as a trained neural network to receive the data streams and produce output based on predefined sets of training data.

In a further aspect, sensors other than event cameras may supply data to the processor and neural network, including other cameras via a feature recognition process.

It is to be understood that both the foregoing general description and the following detailed description are exemplary and explanatory only and should not restrict the scope of the claims. The accompanying drawings, which are incorporated in and constitute a part of the specification, illustrate exemplary embodiments of the inventive concepts disclosed herein and together with the general description, serve to explain the principles.

### BRIEF DESCRIPTION OF THE DRAWINGS

The numerous advantages of the embodiments of the inventive concepts disclosed herein may be better understood by those skilled in the art by reference to the accompanying figures in which:
FIG. 1 shows a block diagram of a system suitable for implementing an exemplary embodiment;
FIG. 2 shows a block diagram of a system according to an exemplary embodiment; and
FIG. 3 shows a block diagram of a neural network according an exemplary embodiment of the inventive concepts disclosed herein.

### DETAILED DESCRIPTION

Before explaining various embodiments of the inventive concepts disclosed herein in detail, it is to be understood that the inventive concepts are not limited in their application to the arrangement of the components or steps or methodologies set forth in the following description or illustrated in the drawings. In the following detailed description of embodiments of the instant inventive concepts, numerous specific details are set forth in order to provide a more thorough understanding of the inventive concepts. However, it will be apparent to one of ordinary skill in the art having the benefit of the instant disclosure that the inventive concepts disclosed herein may be practiced without these specific details. In other instances, well-known features may not be described in detail to avoid unnecessarily complicating the instant disclosure. The inventive concepts disclosed herein are capable of other embodiments or of being practiced or carried out in various ways. Also, it is to be understood that the phraseology and terminology employed herein is for the purpose of description and should not be regarded as limiting.

As used herein a letter following a reference numeral is intended to reference an embodiment of a feature or element that may be similar, but not necessarily identical, to a previously described element or feature bearing the same reference numeral (e.g., 1, 1a, 1b). Such shorthand notations are used for purposes of convenience only, and should not be construed to limit the inventive concepts disclosed herein in any way unless expressly stated to the contrary.

Further, unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of "a" or "an" are employed to describe elements and components of embodiments of the instant inventive concepts. This is done merely for convenience and to give a general sense of the inventive concepts, and "a" and "an" are intended to include one or at least one and the singular also includes the plural unless it is obvious that it is meant otherwise.

Also, while various components may be depicted as being connected directly, direct connection is not a requirement. Components may be in data communication with intervening components that are not illustrated or described.

Finally, as used herein any reference to "one embodiment," or "some embodiments" means that a particular element, feature, structure, or characteristic described in connection with the embodiment is included in at least one embodiment of the inventive concepts disclosed herein. The appearances of the phrase "in at least one embodiment" in the specification does not necessarily refer to the same embodiment. Embodiments of the inventive concepts disclosed may include one or more of the features expressly described or inherently present herein, or any combination or sub-combination of two or more such features.

Broadly, embodiments of the inventive concepts disclosed herein are directed to a wearable device with neuromorphic event cameras. A processor receives data streams from the event cameras and makes application specific predictions / determinations. The event cameras may be outward facing to make determinations about the environment or a specific task, inward facing to monitor the state of the user, or both. The processor may be configured as a trained neural network to receive the data streams and produce output based on predefined sets of training data. Sensors other than event cameras may supply data to the processor and neural network, including other cameras via a feature recognition process.

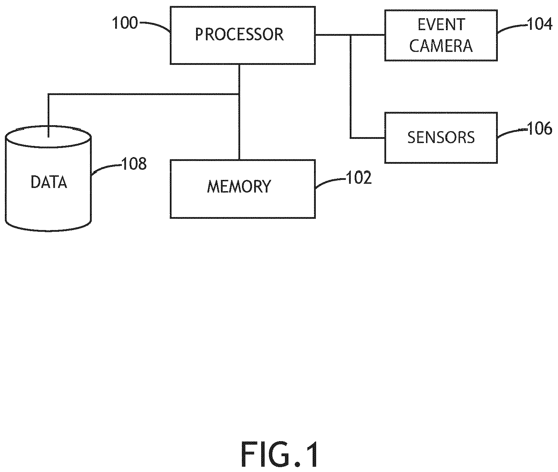

Referring to FIG. 1, a block diagram of a system suitable for implementing an exemplary embodiment is shown. The system, embodied in a wearable device, includes at least one processor 100, memory 102 in data communication with the processor 100 for storing processor executable code, and at least one neuromorphic sensor / event camera 104 in data communication with the processor 100. Event cameras 104 sense changes in light intensity per-pixel; when a change is observed, the pixel is triggered. Event cameras 104 enable low transmission bandwidth, high sampling rate capturing very fast motions, high dynamic range compared to standard frame-based cameras, small size, lightweight, and low power consumption because the event cameras 104 only detect changes and transmit data when there are light changes. Event cameras 104 offer high temporal resolution compared to conventional camera (up to 1 MHz).

In at least one embodiment, the processor 100 is configured to implement an artificial intelligence / machine learning algorithm (e.g., a neural network). Such artificial intelligence / machine learning algorithm is trained to identify lifestyle, state, and health information of the wearer for health monitoring purposes, while overcoming the limitations of RGB cameras. In at least one embodiment, the artificial intelligence / machine learning algorithm is specifically trained to process neuromorphic data without any intermediate conversion. Neural network structures specific to various specific applications may be stored in a data storage element 108, retrieved, and utilized by the processor 100.

In at least one embodiment, the system may include non-image sensors 106 (e.g., trackers, temperature sensors, accelerometers, gyros, galvanic skin sensors, etc.) The processor 100 receives data from those sensors 106 and the artificial intelligence / machine learning algorithms are trained utilize such sensor data to enhance predictions primarily derived from the event cameras 104.

In at least one embodiment, the system includes outward facing event cameras 104 (i.e., affixed to a wearable and pointing toward the environment) and inward facing event cameras 104 (i.e., affixed to a wearable and pointing toward the wearers face). The processor 100 may be trained according to both environmental images and face / eye tracking images.

In at least one embodiment, the processor 100 may receive pixel data and convert them into a RGB space for use with algorithms trained on such RGB data.

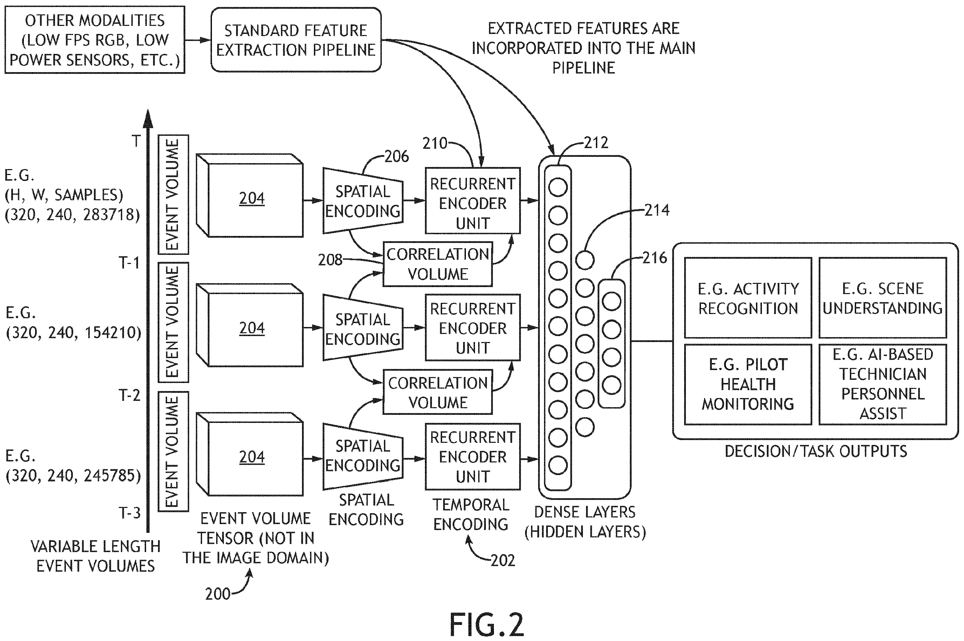

Referring to FIG. 2, a block diagram of a system according to an exemplary embodiment is shown. A wearable system includes one or more event cameras that each produce a data stream 204 of pixel change events. A processor embodying a trained neural network receives the data streams 204 at an input layer 200. Alternatively, the processor may receive the data streams 204 and perform various processing steps prior to supplying data to the neural network.

In at least one embodiment, spatial and temporal encoding layers 202 (or defined processes prior to entering the neural network) receive the data streams 204 and perform spatial encoding 206 to determine and add information about where the corresponding pixels were located in the image. Changes in corresponding pixel locations over time are correlated 208, and recurrent pixel change locations are identified via a recurrent encoder 210. Because the system utilizes event cameras, changes to specific pixels are inherent in the data stream 204.

Based on changing pixel values, correlated over time, hidden layers 212, 214, 216 of the neural network are trained to produce an output for various applications such as activity recognition, object recognition / scene understanding, pilot health monitoring, technical / personal assistance, etc.

In at least one embodiment, event cameras are disposed in a wearable that may be worn on the user's head, creating a first-person perspective. Alternatively, or in addition, the event cameras may be disposed in a wearable on the user's wrist. Both embodiments tend to produce abrupt, unpredictable movement in the resulting image. Event cameras alleviate the problem of such movement and motion blur. Furthermore, embodiments of the present disclosure may include wearables disposed on a user's chest, waist, ankle, or the like. It may be appreciated that wearables disposed anywhere one the user's body are envisioned.

In addition, event cameras may be disposed to observe the wearer's face / eyes. In at least one embodiment, one or more event cameras may comprise an omnidirectional camera configured and disposed to be both outward facing and inward facing.

In at least one embodiment, the neural network may utilize the data streams 204 to estimate motions based on the known disposition of the event cameras on a corresponding wearable. Alternatively, or in addition, the neural network may perform activity recognition. In at least one embodiment, event cameras disposed to observe the wearer's face / eyes may be used by the neural network for health monitoring.

In at least one embodiment, the neural network may receive data from a separate data pipeline 218 configured to identify features via sensors other than event cameras (e.g., tracking sensors, accelerometers, galvanic skin sensors, and the like). The neural network may use data from the separate pipeline 218 to enhance and improve predictions. The system may utilize the separate data pipeline 218 for hand pose estimation; such hand pose estimation may be used in conjunction with the data streams 204 from the event cameras during neural network processing.

The system processes the data streams 204 as streams of event volumes via spatial encoding 206 to extract relevant features and feed those features into a recurring encoder 210 to capture temporal evolution of the data streams 204. Likewise, the system determines how the data streams 204 change in space through correlated volumes. The neural network may then produce a task output specific to a training data set.

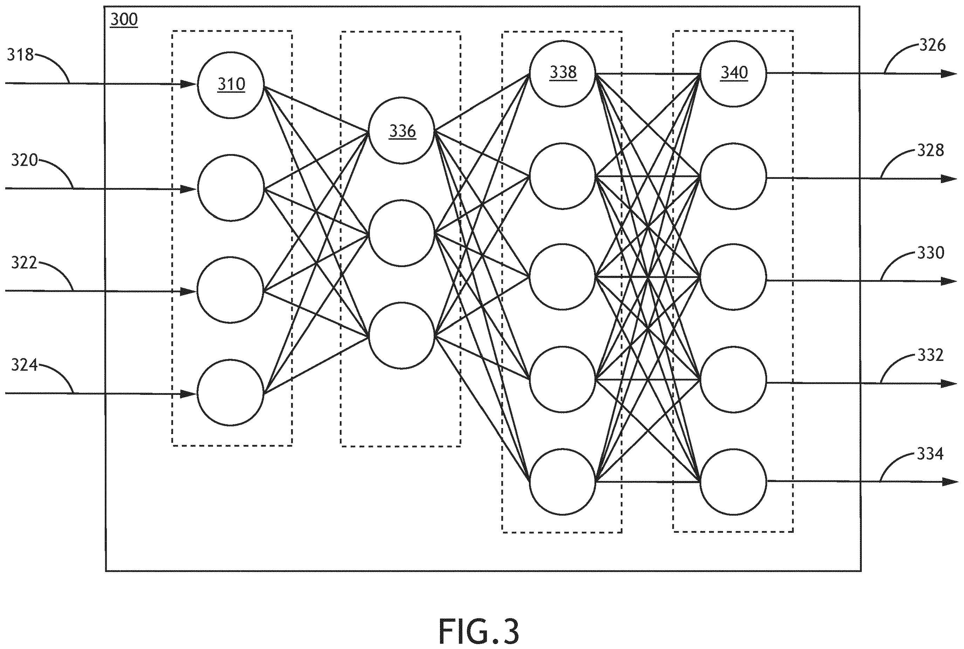

Referring to FIG. 3, a block diagram of a neural network 300 according an exemplary embodiment of the inventive concepts disclosed herein is shown. The neural network 300 comprises an input layer 302, and output layer 304, and a plurality of internal layers 306, 308. Each layer comprises a plurality of neurons or nodes 310, 336, 338, 340. In the input layer 302, each node 310 receives one or more inputs 318, 320, 322, 324 corresponding to a digital signal and produces and output 312 based on an activation function unique to each node 310 in the input layer 302. An activation function may be a Hyperbolic tangent function, a linear output function, and / or a logistic function, or some combination thereof, and different nodes 310, 336, 338, 340 may utilize different types of activation functions. In at least one embodiment, such activation function comprises the sum of each input multiplied by a synaptic weight. The output 312 may comprise a real value with a defined range or a Boolean value if the activation function surpasses a defined threshold. Such ranges and thresholds may be defined during a training process. Furthermore, the synaptic weights are determined during the training process.

Outputs 312 from each of the nodes 310 in the input layer 302 are passed to each node 336 in a first intermediate layer 306. The process continues through any number of intermediate layers 306, 308 with each intermediate layer node 336, 338 having a unique set of synaptic weights corresponding to each input 312, 314 from the previous intermediate layer 306, 308. It is envisioned that certain intermediate layer nodes 336, 338 may produce a real value with a range while other intermediated layer nodes 336, 338 may produce a Boolean value. Furthermore, it is envisioned that certain intermediate layer nodes 336, 338 may utilize a weighted input summation methodology while others utilize a weighted input product methodology. It is further envisioned that synaptic weight may correspond to bit shifting of the corresponding inputs 312, 314, 316.

An output layer 304 including one or more output nodes 340 receives the outputs 316 from each of the nodes 338 in the previous intermediate layer 308. Each output node 340 produces a final output 326, 328, 330, 332, 334 via processing the previous layer inputs 316. Such outputs may comprise separate components of an interleaved input signal, bits for delivery to a register, or other digital output based on an input signal and DSP algorithm.

In at least one embodiment, each node 310, 336, 338, 340 in any layer 302, 306, 308, 304 may include a node weight to boost the output value of that node 310, 336, 338, 340 independent of the weighting applied to the output of that node 310, 336, 338, 340 in subsequent layers 304, 306, 308. It may be appreciated that certain synaptic weights may be zero to effectively isolate a node 310, 336, 338, 340 from an input 312, 314, 316, from one or more nodes 310, 336, 338 in a previous layer, or an initial input 318, 320, 322, 324.

In at least one embodiment, the number of processing layers 302, 304, 306, 308 may be constrained at a design phase based on a desired data throughput rate. Furthermore, multiple processors and multiple processing threads may facilitate simultaneous calculations of nodes 310, 336, 338, 340 within each processing layers 302, 304, 306, 308.

Layers 302, 304, 306, 308 may be organized in a feed forward architecture where nodes 310, 336, 338, 340 only receive inputs from the previous layer 302, 304, 306 and deliver outputs only to the immediately subsequent layer 304, 306, 308, or a recurrent architecture, or some combination thereof.

In at least one embodiment, initial inputs 318, 320, 322, 324 may comprise any sensor input from one or more wearable event cameras. Final output 326, 328, 330, 332, 334 may comprise object recognition data, user health data, or the like.

Embodiments of the present disclosure are useful for low light scenarios and small, lightweight, low power consumption wearables.

It is believed that the inventive concepts disclosed herein and many of their attendant advantages will be understood by the foregoing description of embodiments of the inventive concepts, and it will be apparent that various changes may be made in the form, construction, and arrangement of the components thereof without departing from the broad scope of the inventive concepts disclosed herein or without sacrificing all of their material advantages; and individual features from various embodiments may be combined to arrive at other embodiments. The forms herein before described being merely explanatory embodiments thereof, it is the intention of the following claims to encompass and include such changes. Furthermore, any of the features disclosed in relation to any of the individual embodiments may be incorporated into any other embodiment.

## Claims

1. A computer apparatus in a wearable device comprising:
at least one event camera (104), at least one of the event cameras (104) disposed to be outwardly facing; and
at least one processor (100) in data communication with the at least one event camera (104) and a memory storing processor executable code for configuring the at least one processor (100) to:
receive data streams from the at least one event camera (104), each data stream comprising one or more pixel change events;
associate the one or more pixel change events to an environmental event; and
produce a determination about the environmental event based on a known data set.

2. The computer apparatus of Claim 1, wherein the at least one processor (100) is configured to instantiate a neural network to produce the determination about the environmental event from an input layer receiving the data streams.

3. The computer apparatus of Claim 1 or 2, wherein the at least one processor (100) is configured to:
perform spatial encoding of the data streams; and
perform recurrent encoding of the data stream, and optionally wherein the at least one processor (100) is configured to correlate event volumes for spatial encoding.

4. The computer apparatus of any preceding Claim, further comprising at least one non-image sensors, wherein:
the at least one processor (100) is further configured to:
receive data streams from the at least one non-image sensors; and
correlate the data streams from the non-image sensor with the data streams from the event cameras (104); and
producing the determination about the environmental event is based on both the data streams from the non-image sensor and the data streams from the event cameras (104).

5. The computer apparatus of Claim 4, wherein:
at least one of the event cameras (104) is disposed to be inwardly facing; and
the at least one processor (100) is further configured to:
receive one or more data streams from the at least one inwardly facing event camera (104);
correlate the outward facing event camera data streams and inwardly facing event camera data streams via temporal encoding; and
produce a determination about a user health state.

6. The computer apparatus of Claim 4 or 5, wherein the at least one non-image sensor comprises a temperature sensor, accelerometer, gyro, or galvanic skin sensor.

7. An wearable event monitoring system comprising:
at least one event camera (104), at least one of the event cameras (104) disposed to be outwardly facing; and
at least one processor (100) in data communication with the at least one event camera (104) and a memory storing processor executable code for configuring the at least one processor (100) to:
receive data streams from the at least one event camera (104), each data stream comprising one or more pixel change events;
associate the one or more pixel change events to an environmental event; and
produce a determination about the environmental event based on a known data set.

8. The system of Claim 7, wherein the at least one processor (100) is configured to instantiate a neural network to produce the determination about the environmental event from an input layer receiving the data streams.

9. The system of Claim 7 or 8, wherein the at least one processor (100) is configured to:
perform spatial encoding of the data streams; and
perform recurrent encoding of the data stream, and optionally wherein the at least one processor (100) is configured to correlate event volumes for spatial encoding.

10. The system of any of Claims 7 to 9, further comprising at least one non-image sensors, wherein:
the at least one processor (100) is further configured to:
receive data streams from the at least one non-image sensors; and
correlate the data streams from the non-image sensor with the data streams from the event cameras (104); and
producing the determination about the environmental event is based on both the data streams from the non-image sensor and the data streams from the event cameras (104).

11. The system of Claim 10, wherein:
at least one of the event cameras (104) is disposed to be inwardly facing; and
the at least one processor (100) is further configured to:
receive one or more data streams from the at least one inwardly facing event camera (104);
correlate the outward facing event camera data streams and inwardly facing event camera data streams via temporal encoding; and
produce a determination about a user health state, and/or wherein the at least one non-image sensor comprises a temperature sensor, accelerometer, gyro, or galvanic skin sensor in data communication with a separate data pipeline.

12. A wearable comprising:
at least one event camera (104), at least one of the event cameras (104) disposed to be outwardly facing; and
at least one processor (100) in data communication with the at least one event camera (104) and a memory storing processor executable code for configuring the at least one processor (100) to:
receive data streams from the at least one event camera (104), each data stream comprising one or more pixel change events;
associate the one or more pixel change events to an environmental event; and
produce a determination about the environmental event based on a known data set.

13. The wearable of Claim 12, wherein the at least one processor (100) is configured to instantiate a neural network to produce the determination about the environmental event from an input layer receiving the data streams.

14. The wearable of Claim 12 or 13, wherein the at least one processor (100) is configured to:
perform spatial encoding of the data streams; and
perform recurrent encoding of the data stream, and optionally wherein the at least one processor (100) is configured to correlate event volumes for spatial encoding.

15. The wearable of any of Claims 12 to 14, further comprising at least one non-image sensors, wherein:
the at least one processor (100) is further configured to:
receive data streams from the at least one non-image sensors; and
correlate the data streams from the non-image sensor with the data streams from the event cameras (104); and
producing the determination about the environmental event is based on both the data streams from the non-image sensor and the data streams from the event cameras (104), and optionally wherein:
at least one of the event cameras (104) is disposed to be inwardly facing; and
the at least one processor (100) is further configured to:
receive one or more data streams from the at least one inwardly facing event camera;
correlate the outward facing event camera data streams and inwardly facing event camera data streams via temporal encoding; and
produce a determination about a user health state.
